# EUROPÄISCHE PATENTSCHRIFT

(11) **EP 3 331 470 B1**
(45) Veröffentlichungstag und Bekanntmachung des Hinweises auf die Patenterteilung: **15.07.2020**
(21) Anmeldenummer: 17791569.1
(22) Anmeldetag: 19.09.2017
(51) Int. Cl.: A61C 3/02, A61C 8/00, A61B 17/16

(54) **CHIRURGISCHES INSTRUMENT**
SURGICAL INSTRUMENT
INSTRUMENT CHIRURGICAL

(30) Priorität: 31.10.2016 DE 102016120755
(43) Veröffentlichungstag der Anmeldung: 13.06.2018
(73) Patentinhaber: Zastrow, Frank, 69120 Heidelberg (DE)
(72) Erfinder: Zastrow, Frank, 69120 Heidelberg (DE)
(74) Vertreter: Patent- und Rechtsanwälte Ullrich & Naumann
(86) Internationale Anmeldenummer: PCT/DE2017/200097
(87) Internationale Veröffentlichungsnummer: WO 2018/077354

(56) Entgegenhaltungen:
- EP-A2- 0 083 558
- WO-A1-2008/088105
- WO-A1-2011/026164
- US-A1- 2008 249 553

## Beschreibung

Die vorliegende Erfindung betrifft ein chirurgisches Instrument zur Anwendung in der Dentalchirurgie.

Chirurgisch tätige Zahnärzte, Oralchirurgen und Kieferchirurgen stehen häufig vor der Herausforderung, dass durch Knochenatrophie, durch Unfälle, durch Parodontitis oder durch Zahnextraktion Knochen in der Mundhöhle verloren gegangen ist.

Wenn Zahnimplantate zum Einsatz neuer Zähne geplant werden, so ist es wichtig, dass diese Knochendefizite vorher oder simultan mit der Implantatsetzung aufgebaut werden, damit die Zahnimplantate wieder ein neues Fundament und eine stabile Abstützung im Knochen haben.

Autologer, patienteneigener Knochen gilt dabei nach wie vor als Goldstandard bei knochenaufbauenden Eingriffen. Dies liegt an den Eigenschaften des Knochens, da autologer Knochen sowohl osteogene, osteoinduktive als auch osteokonduktive Eigenschaften miteinander vereint. Das bedeutet, der Knochen hat die Potenz, eigenes Knochengewebe zu bilden, Gefäße zu bilden und wirkt zudem als Leitstruktur für neu gebildeten Knochen. Knochenersatzmaterial hat im Gegensatz zu autologem Knochen keine biologische Potenz und wirkt lediglich osteokonduktiv, das heißt, es wirkt auch als Leitschiene.

Beim Arbeiten mit autologem Knochen sind verschiedene Verfahren vorbekannt. Bei größeren Knochendefiziten bieten sich als zweite intraorale Entnahmestelle grundsätzlich zahnlose Knochenareale an, alternativ der Tuber maxillae, die Spina nasalis anterior, der Gaumen, der Bereich der Kieferhöhlenwand im Oberkiefer oder aber der Unterkiefer, da dieser eher kortikaler Natur ist und die Knochenqualität als sehr gut und stabil gilt. Im Unterkiefer gibt es verschiedene Knochenentnahmestellen, so beispielsweise wiederum zahnlose Areale, das Kinn oder den retromolare Bereich.

Die Knochenentnahme kann hierbei mit verschiedenen Instrumenten erfolgen. Das Konzept der Knochenentnahme ist dabei meist ähnlich.
Entweder werden mit einer sogenannten Lindemannfräse oder mit einem Piezosurgerygerät oder aber mit einer kleinen Säge drei bis vier Sollbruchstellen geschaffen und der Block im Nachhinein mit einem Meisel oder einem anderen Instrument herausgebrochen. Dabei ist nachteilig, dass bei einer Knochenentnahme mehr oder weniger große Gewaltanwendung auf den Kiefer nötig ist. Daher wird dieser Eingriff teilweise vom Arzt gescheut, auch aus dem Grund, da dieses Heraushämmern bzw. -brechen des Knochenblockes aus der jeweiligen Region auch für den Patienten unangenehm ist.

Zahnärzte sind aufgrund Ihres Berufes rotierende Instrumente und Bohrer gewohnt. So hat sich auch die sogenannte Trepanfräse etabliert, die an ein Handstück angesteckt wird und einen Kopf aufweist, der als Hohlzylinder ausgebildet ist. An dem distalen Rand des Kopfes sind Zähne zur spanenden Bearbeitung des Knochens ausgebildet. Trepanfräsen werden beispielsweise zur Implantatbettaufbereitung genutzt und weisen einen Durchmesser von ca. 3 mm bis 4 mm auf. Mit Hilfe dieser Fräsen werden äußerst kleine und schmale Bohrzylinder entnommen, die nur bedingt zum Knochenaufbau geeignet sind.

Bei den voranstehend genannten Vorrichtungen müssen zur Knochenentnahme ca. drei bis vier Schnitte bzw. Sollbruchstellen angelegt werden, um das benötigte Knochenstück zu erhalten. Dies bedingt eine starke Belastung für den Patienten und erfordert großes handwerkliches Geschick durch den Operateur. Insbesondere ist dabei zu beachten, dass das umliegende Weichgebewebe, beispielsweise Wange oder Lippe, durch das chirurgische Werkzeug nicht verletzt wird.

Weitere Ausgestaltungen von Trepanfräsen sind in den Dokumenten WO 2008/088105 A1, WO 2011/026164 A1 und EP 0 083 558 A1 beschrieben. Das Dokument US 2008/0249553 A1 betrifft eine Vorrichtung zum Entfernen eines Myoms an einer Gebärmutter.

Der Erfindung liegt daher die Aufgabe zugrunde, ein chirurgisches Instrument derart auszugestalten und weiterzubilden, dass mit konstruktiv einfachen Mitteln eine zuverlässige und für den Patienten schonende Knochenentnahme möglich ist.

Erfindungsgemäß wird die voranstehende Aufgabe durch die Merkmale des Anspruchs 1 gelöst. Danach ist ein chirurgisches Instrument zur Anwendung in der Dentalchirurgie, mit einer Bearbeitungseinrichtung und einer Schutzeinrichtung angegeben, wobei die Bearbeitungseinrichtung einen Schaft mit einem Anschlussbereich und einem hohlzylinderförmigen Kopf aufweist, wobei an einem distalen Rand des Kopfes ein Wirkbereich zum spanenden oder schleifenden Bearbeiten von Knochen, ausgebildet ist, wobei die Schutzeinrichtung den Kopf zumindest bereichsweise umgibt, wobei die Schutzeinrichtung den Kopf derart umgibt, dass lediglich ein Kreisbogen des distalen Randes als Wirkbereich dient und wobei die Schutzeinrichtung mit einem sich zumindest bereichsweise innerhalb des Kopfes erstreckenden Lagerelement der Bearbeitungseinrichtung drehbar gekoppelt ist.

In erfindungsgemäßer Weise ist erkannt worden, dass die zugrundeliegende Aufgabe durch die Kombination einer Bearbeitungseinrichtung, insbesondere einer Trepanfräse, und einer Schutzeinrichtung in verblüffend einfacher Weise gelöst werden kann. Dazu weist die Bearbeitungseinrichtung einen Schaft mit einem Anschlussbereich und einem hohlzylinderförmigen Kopf auf. Der Anschlussbereich kann zur Verbindung mit einem chirurgischen Handgerät, beispielsweise einem gängigen Winkelstück oder Handstück dienen, wie es von Zahnärzten bzw. Dentalchirurgen verwendet wird. Des Weiteren ist der distale Rand des Kopfes als Wirkbereich ausgebildet, insbesondere zum spanenden Bearbeiten von Knochen. Beispielsweise kann der distale Rand Schneide- bzw. Sägezähnen und/oder eine Diamantierung aufweisen.

In weiter erfindungsgemäßer Weise ist erkannt worden, dass die Schutzeinrichtung auf besonders sichere und einfache Weise mit dem Kopf bzw. der Bearbeitungseinrichtung drehbar gekoppelt werden kann, indem die Bearbeitungseinrichtung ein zumindest bereichsweise innerhalb des Kopfes verlaufendes Lagerelement aufweist. Somit ist es möglich, dass mit dem distalen Rand der von einem medizinischen Handgerät rotierten Bearbeitungseinrichtung Knochen an der Entnahmestelle bearbeitet wird, während die Schutzeinrichtung zumindest im Wesentlichen ortsfest bleibt, nämlich aufgrund der drehbaren Verbindung zwischen Schutzeinrichtung und Bearbeitungseinrichtung. Somit ist das umliegende Gewebe in idealer Weise von Verletzungen geschützt.

Dabei ist im Konkreten denkbar, dass sich das Lagerelement in Axialrichtung, insbesondere von dem Boden des Kopfes, zumindest bis zu dem distalen Rand des Kopfes erstreckt. Die Schutzeinrichtung kann an dem freien Ende des Lagerelements angekoppelt sein. Das Lagerelement muss in Axialrichtung nicht zwangsweise bis zu dem distalen Rand verlaufen, sofern die Schutzeinrichtung entsprechend weit in den Kopf hineinragt, um drehbar mit dem Lagerelement gekoppelt zu sein. In weiter vorteilhafter Weise erstreckt sich das Lagerelement in Axialrichtung bis über den distalen Rand des Kopfes hinweg, so dass die Verbindung zwischen Schutzeinrichtung und Bearbeitungseinrichtung außerhalb des Kopfes realisiert ist. Im Konkreten ist dabei denkbar, dass das Lagerelement zapfenförmig ausgebildet ist und in einen korrespondierenden Bereich der Schutzeinrichtung eingreift.

In erfindungsgemäßer Weise umgibt die Schutzeinrichtung den Kopf derart, dass lediglich ein Kreisbogen des distalen Randes als Wirkbereich dient. Entgegen eines Vorurteils der Fachwelt ist dabei erkannt worden, dass eine Bearbeitungseinrichtung mit einem als Hohlzylinder ausgebildeten Kopf, der einen Wirkbereich zur spanenden Bearbeitung von Knochen aufweist, sich nicht nur zur Entnahme von kleinen Bohrzylindern eignet. Vielmehr lässt sich eine entsprechend ausgebildete Bearbeitungseinrichtung dazu nutzen, das benötigte Knochenstück bzw. Knochensegment aus den Knochen "herauszuschälen". Durch die besondere Anordnung und Ausgestaltung der Schutzeinrichtung ist lediglich ein Kreisbogen des distalen Randes des Kopfes mit dem Knochen in Kontakt bringbar und dient somit als effektiver Wirkbereich. Durch diese konstruktive Maßnahme ist es den Operateuren möglich, ein etwa halbmondförmiges Knochenstück aus dem Knochen "abzuschälen", das für den Knochenaufbau verwendbar ist. Im Gegensatz zu den aus dem Stand der Technik bekannten Instrumenten bzw. Werkzeugen müssen keine drei bis vier Schnitte bzw. Sollbruchstellen angelegt werden. Es entsteht vielmehr lediglich eine einzige Sollbruchstelle - bspw. apikal -, ein Heraushämmern des Knochens ist nicht notwendig. Der entstehende Block ist sodann ohne größere Gewalteinwirkung herauslösbar bzw. luxierbar, was für den Patienten im Vergleich zu den bekannten Vorrichtungen und Techniken sehr viel schonender und angenehmer ist. In vorteilhafter Weise umgibt die Schutzeinrichtung den Kopf derart, dass ein von dem Kreisbogen gebildetes Kreissegment eine Segmenthöhe von 2,0 mm bis 3,5 mm, vorzugsweise von 2,2 mm bis 2,5 mm, aufweist.

Insbesondere ist denkbar, dass der Kopf einen Innendurchmesser von 5 mm bis 10 mm, vorzugsweise von 6 mm bis 8 mm, und/oder eine Wanddicke von 0,2 mm bis 0,6 mm, vorzugsweise von 0,4 mm aufweist. Alternativ oder zusätzlich kann der Kopf eine Tiefe von 6 mm bis 17 mm, insbesondere von 9 mm bis 14 mm aufweisen und/oder das Lagerelement eine Länge von 10 bis 19 mm, insbesondere von 12 bis 17 mm aufweisen. In besonders vorteilhafter Weise kann sich das Lagerelement in Axialrichtung gesehen 2 mm bis 4 mm, insbesondere 3 mm, über den distalen Rand des Kopfes hinaus erstrecken.

Um das distale Ende der Schutzeinrichtung zum Verdrängen des umliegenden Gewebes an der Entnahmestelle zu verwenden, kann die Schutzeinrichtung in bevorzugter Weise an ihrem distalen Ende vollständig, zumindest jedoch weitgehend, geschlossen ausgebildet sein. Dabei wird ein besonderer kombinatorischer Effekt mit der Ausgestaltung des Lagerelements erzielt, welches nämlich die Schutzeinrichtung stabilisiert, wenn das außenliegende Gewebe auf das distale Ende der Schutzeinrichtung einen Druck, insbesondere in Axialrichtung, ausübt.

Um eine sichere Verbindung zwischen der Schutzeinrichtung und der Bearbeitungseinrichtung herzustellen, kann die Schutzeinrichtung an ihrem proximalen Ende mit dem Schaft drehbar gekoppelt sein. Im Konkreten ist es hierbei denkbar, dass die Schutzeinrichtung den Schaft in Umfangsrichtung umgibt.

In vorteilhafter Weise kann die Schutzeinrichtung über ein Gleitlager und/oder ein Kugellager und/oder ein Wälzlager mit dem Lagerelement und/oder dem Schaft drehbar gekoppelt sein. Insbesondere ein Gleitlager ist hierbei von Vorteil, da sich dieses durch eine extrem einfache und somit kostengünstige Konstruktion auszeichnet. Das Gleitlager muss dabei nicht als separates Element ausgebildet sein, vielmehr kann der entsprechende Bereich der Schutzeinrichtung bzw. der Bearbeitungseinrichtung das Gleitlager darstellen.

Um zu verhindern, dass das bearbeitete Gewebe durch ein Überhitzung Schaden nimmt, kann an dem Lagerelement mindestens eine Fluidöffnung, beispielsweise mit einem Durchmesser von ca. 0,5 mm bis 0,9 mm, vorzugsweise 0,7 mm, ausgebildet sein, die mit einem Fluidkanal, beispielsweise mit einem Durchmesser von ca. 0,6 mm bis 1,0 mm, vorzugsweise von 0,8 mm, in Verbindung steht. Der Fluidkanal kann sich von dem Anschlussbereich durch den Schaft und das Lagerelement hindurch zu der mindestens einen Fluidöffnung erstrecken. Somit kann eine Kühlflüssigkeit beispielsweise von dem chirurgischen Handstück, mit dem das chirurgische Instrument über den Anschlussbereich verbunden ist, eingebracht werden. Da das Lagerelement innerhalb des hohlzylinderförmigen Kopfes angeordnet ist, können der hohlzylinderförmige Kopf und insbesondere der distale Rand in idealer Weise mit Kühlflüssigkeit beaufschlagt werden.

In weiter vorteilhafter Weise kann die Schutzeinrichtung aus Kunststoff oder Metall hergestellt sein. Kunststoff hat den Vorteil, dass die Schutzeinrichtung besonders einfach und somit preisgünstig herstellbar ist. Des Weiteren kann auf besonders einfache Weise ein Gleitlager zur Verbindung der Schutzeinrichtung mit dem Lagerelement und/oder dem Schaft erzeugt werden, wenn die Schutzeinrichtung aus Kunststoff gefertigt ist. Die Realisierung der Schutzeinrichtung aus Metall bietet den Vorteil, dass die Schutzeinrichtung wieder aufbereitet werden kann - beispielsweise in einem Autoklaven - so dass eine Mehrfachverwendung möglich ist.

Um dem Operateur eine gute Sicht auf die Entnahmestelle zu ermöglichen, kann die Schutzeinrichtung in idealer Weise transparent - beispielsweise aus einem durchsichtigen Kunststoff - ausgebildet sein. Alternativ oder zusätzlich kann an der Außenwand des Kopfes eine Tiefenmarkierung ausgebildet sein, so dass der Operateur auf einfache Weise nachvollziehen kann, wie tief der Kopf in den zu bearbeitenden Knochen eingebracht ist. Beispielsweise kann hierzu in regelmäßigen Abständen ein, insbesondere schwarzer, Streifen, vorzugsweise als Lasermarkierung, an der Außenwand ausgebildet sein. Die Tiefenmarkierung kann insbesondere in Schritten von 1 mm bis 3 mm, vorzugweise von 2 mm, ausgebildet sein.

In besonders raffinierter Weise kann in der Seitenwand des Kopfes zumindest eine Öffnung ausgebildet sein, durch welche der Operateur das entnommene Knochenstück - beispielsweise mit einer Pinzette - aus dem Kopf herausschieben kann. Die Öffnung kann beispielsweise oval ausgebildet sein, wobei die längste Achse insbesondere 3 mm bis 5 mm, vorzugweise 4 mm betragen kann.

In weiter vorteilhafter Weise kann an dem Schaft ein Sicherungselement aus einem Werkstoff, insbesondere aus Kunststoff, ausgebildet sein, dessen Form und/oder Farbe sich bei einer Aufbereitung des Instruments, beispielsweise beim Autoklavieren, verändert. In besonders vorteilhafter Weise kann das Sicherungselement im oder am Anschlussbereich angeordnet sein und sich bei einem Aufbereiten des Instruments derart verändern, dass das Instrument nicht mehr mit einem medizinischen Handgerät gekoppelt werden kann.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen, perspektivischen Darstellung ein Ausführungsbeispiel eines erfindungsgemäßen chirurgischen Instruments,
- Fig. 2: in einer schematischen, seitlichen Darstellung das erfindungsgemäße chirurgische Instrument gemäß Fig.1,
- Fig. 3: in einer weiteren schematischen, perspektivischen Darstellung das erfindungsgemäße chirurgische Instrument gemäß Fig.1,
- Fig. 4: in einer schematischen, geschnittenen Darstellung das erfindungsgemäße chirurgische Instrument gemäß Fig.1,
- Fig. 5: in einer weiteren schematischen, geschnittenen Darstellung das erfindungsgemäße chirurgische Instrument gemäß Fig.1,
- Fig. 6: in einer schematischen, teilweise geschnittenen Darstellung das erfindungsgemäße chirurgische Instrument gemäß Fig.1,
- Fig. 7: in einer weiteren schematischen, teilweise geschnittenen Darstellung das erfindungsgemäße chirurgische Instrument gemäß Fig.1,
- Fig. 8: in einer weiteren schematischen, teilweise geschnittenen Darstellung das erfindungsgemäße chirurgische Instrument gemäß Fig.1,
- Fig. 9: in einer schematischen, perspektivischen Explosionsdarstellung das erfindungsgemäße chirurgische Instrument gemäß Fig.1, und
- Fig. 10: in einer weiteren schematischen, perspektivischen Explosionsdarstellung das erfindungsgemäße chirurgische Instrument gemäß Fig.1.

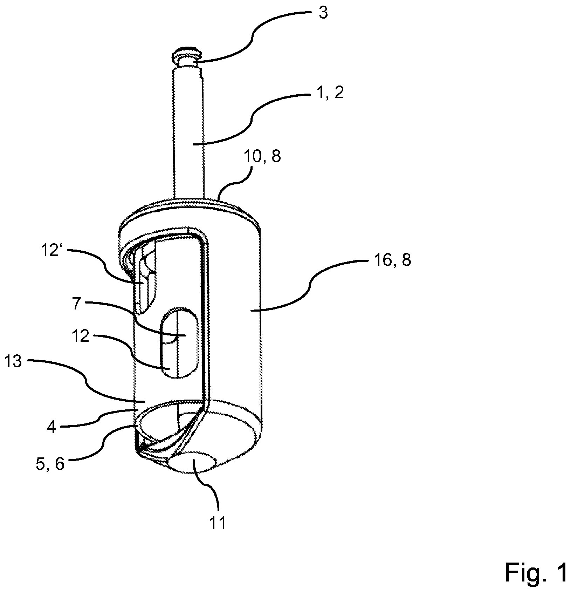

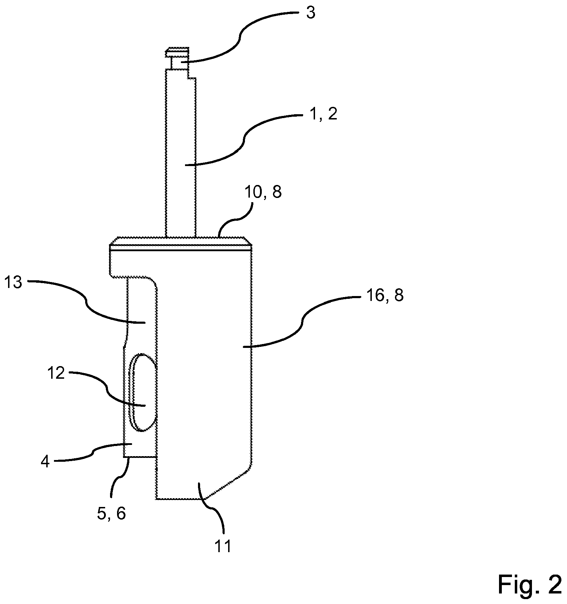

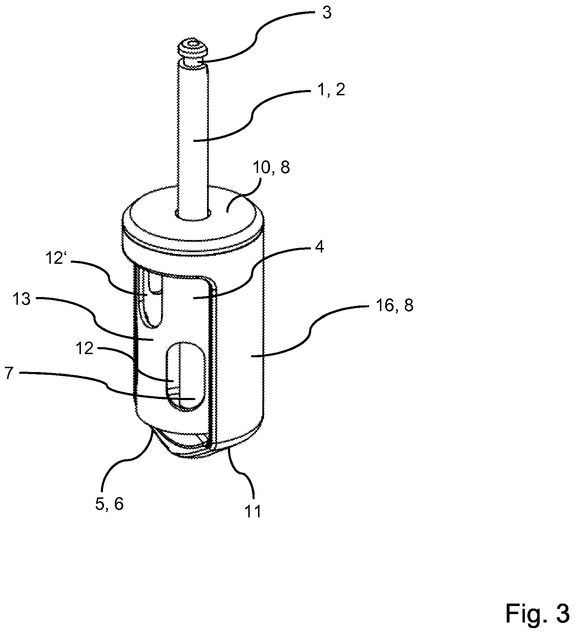

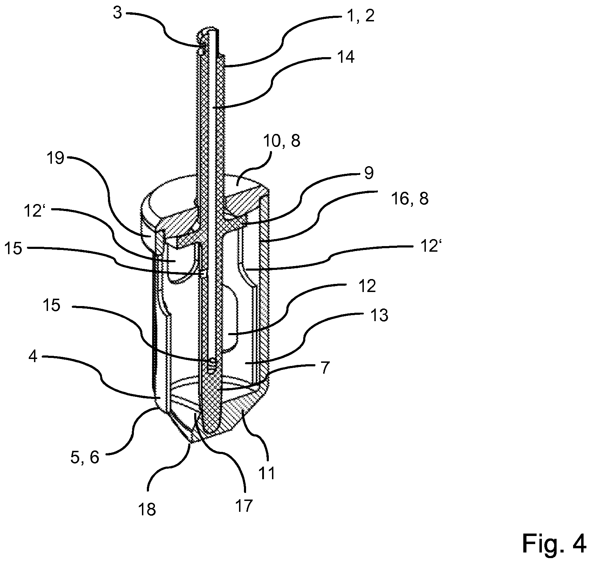

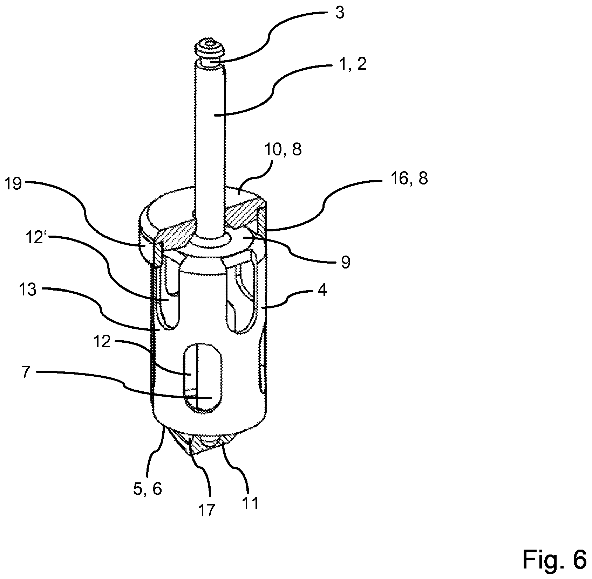

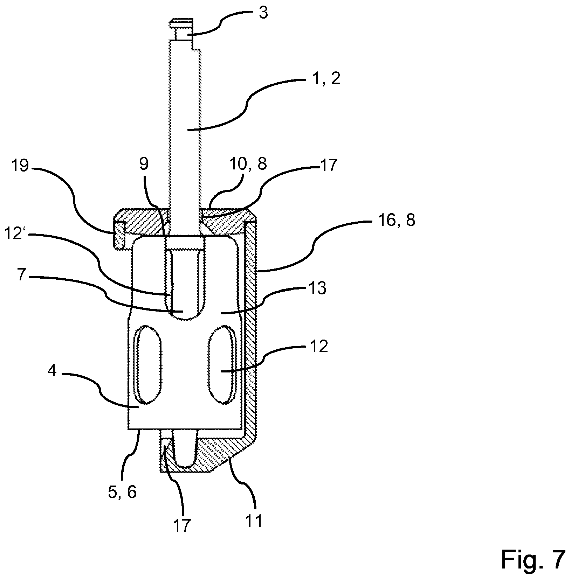

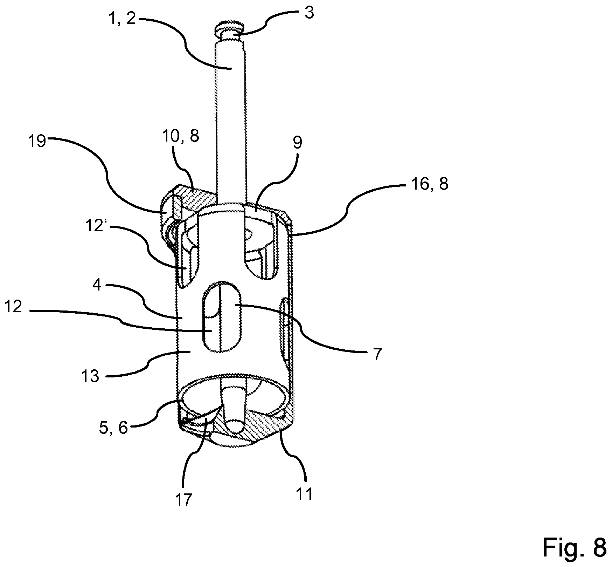

Die Figuren 1 bis 10 zeigen in verschiedenen Darstellungen ein Ausführungsbeispiel eines erfindungsgemäßen chirurgischen Instruments. Das chirurgische Instrument umfasst eine Bearbeitungseinrichtung 1, die im hier dargestellten Ausführungsbeispiel als Hohlfräse bzw. Trepanfräse ausgebildet ist. Die Bearbeitungseinrichtung 1 umfasst einen Schaft 2, der einen Anschlussbereich 3 zum Ankoppeln an ein nicht dargestelltes chirurgisches Handgerät, beispielsweise ein Winkelstück oder Handstück eines Zahnarztes bzw. Dentalchirurgen, aufweist. Über das Handgerät kann die Bearbeitungseinrichtung 1 in Rotation versetzt werden.

An dem Schaft 2 ist ein hohlzylinderförmiger Kopf 4 angeordnet. Der Kopf 4 weist einen distalen Rand 5 auf, der als Wirkbereich 6 zur Bearbeitung bzw. zum Herauslösen des Knochens dient. Beispielsweise können an dem distalen Rand 5 eine Diamantierung und/oder Säge- bzw. Schneidzähnen ausgebildet sein.

Innerhalb des Kopfes 4 verläuft ein Lagerelement 7, das als Lagerzapfen ausgebildet ist. Im hier dargestellten Ausführungsbeispiel erstreckt sich das Lagerelement 7 von dem Boden 9 des Kopfes 4 in Axialrichtung durch den gesamten Kopf 4 bis über den distalen Rand 5 hinweg. Im Konkreten ist das Lagerelement 7 als Verlängerung des Schaftes 2 ausgebildet.

Des Weiteren ist eine Schutzeinrichtung 8 drehbar mit der Bearbeitungseinrichtung 1 gekoppelt. Der Boden 10 der Schutzeinrichtung umgibt den Schaft 2 und ist sowohl mit dem Schaft 4 als auch mit dem Boden 9 des Kopfes 4 drehbar gekoppelt, kann nämlich das Lagerelement 7 in einer Ausnehmung des distalen Endes 11 angeordnet sein. Des Weiteren ist das distale Ende 11 der Schutzeinrichtung 8 geschlossen ausgebildet und ist die Schutzeinrichtung 8 über das distale Ende 11 mit dem Lagerelement 7 drehbar gekoppelt. An dieser Stelle sei darauf hingewiesen, dass die Kopplung zwischen Schutzeinrichtung 8 und Bearbeitungseinrichtung 1 auch über ein separates Lager erfolgen kann. Wesentlich ist lediglich, dass mit dem rotierenden Kopf 4 der Bearbeitungseinrichtung 1 der Knochen bearbeitet bzw. herausgelöst werden kann, während sich die Schutzeinrichtung 8 im Wesentlichen nicht dreht und das umliegende Gewebe schützt.

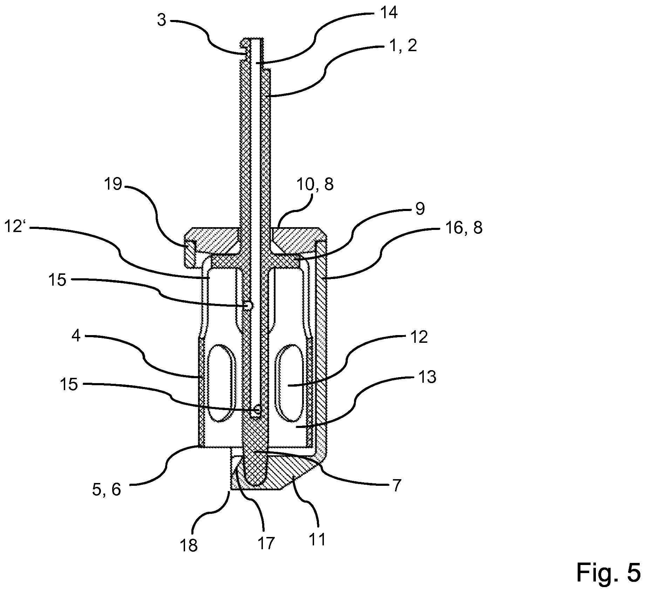

Insbesondere aus Fig. 1 geht deutlich hervor, dass die Schutzeinrichtung 8 die Bearbeitungseinrichtung 1 derart umgibt, dass lediglich ein Kreisbogen des distalen Randes 5 als Wirkbereich 6 dient bzw. mit dem zu bearbeitenden Knochen in Kontakt bringbar ist. Durch diese Maßnahme wird ein zu tiefes Eindringen in den Knochen verhindert sowie umliegendes Gewebe geschützt. Ferner wird es dem Operateur ermöglicht, ein etwa halbmondförmiges Knochenstück aus dem Kiefer herauszuschälen. Durch die Kopplung des distalen Endes 11 der Schutzeinrichtung 8 mit dem Lagerelement 7 kann das distale Ende 11 als Verdrängerelement dienen, mit welchem das umliegende Gewebe während des Eingriffs wegschiebbar ist. Insbesondere in Fig. 5 ist deutlich zu erkennen, dass an dem distalen Ende 11 eine Schräge 17 ausgebildet ist. Durch die Schräge 17 ist das Entfernen des Knochensegments aus dem Kopf 4 vereinfacht. Damit das distale Ende 11 im Bereich der Schräge 17 massiv genug ausgebildet ist, verringert sich der Durchmesser des Lagerelements 7 an seinem Ende. Des Weiteren sei darauf hingewiesen, dass der Eckbereich 18 des distalen Endes 11 abgerundet ausgebildet sein kann, um ein Verkanten der Schutzeinrichtung 8 zu verhindern.

Um das kreissegmentförmige Knochenstück aus dem Kopf 4 zu entfernen, sind Öffnungen 12 in der Seitenwand 13 des Kopfes 4 ausgebildet. Der Operateur kann somit das ausgelöste Knochenstück durch die Öffnung 12 - beispielsweise mit einer Pinzette oder einem ähnlichen Werkzeug - aus dem Kopf 4 herausschieben. Aus Fig. 5 geht deutlich hervor, dass die sich die Öffnung 12' bis zu dem Boden 9 des Kopfes erstreckt, so dass das entnommene Knochensegment auf besonders einfache Weise mit einem separaten Instrument aus dem Kopf 4 herausgeschoben werden kann. Um dies nochmals zu vereinfachen, schließt der Rand 19 des Zylinderelements 16 in Axialrichtung im Wesentlichen mit dem Boden 9 des Kopfes 4 ab.

Um die Eingriffsstelle zu kühlen, ist ein Fluidkanal 14 ausgebildet, der sich von dem Anschlussbereich 3 durch den Schaft 4 und das Lagerelement 7 hindurch erstreckt. Insbesondere Fig. 5 ist deutlich zu entnehmen, dass in dem Lagerelement 7 zwei Fluidöffnungen 15 ausgebildet sind, die mit dem Fluidkanal 14 in Verbindung stehen, so dass Kühlflüssigkeit von dem chirurgischen Handstück über den Anschlussbereich 3 und den Fluidkanal 14 zu den Fluidöffnungen 15 förderbar ist. Die Kühlflüssigkeit wird von den Fluidöffnungen 15 auf den Kopf 4 und/oder die dahinterliegende Entnahmestelle gesprüht.

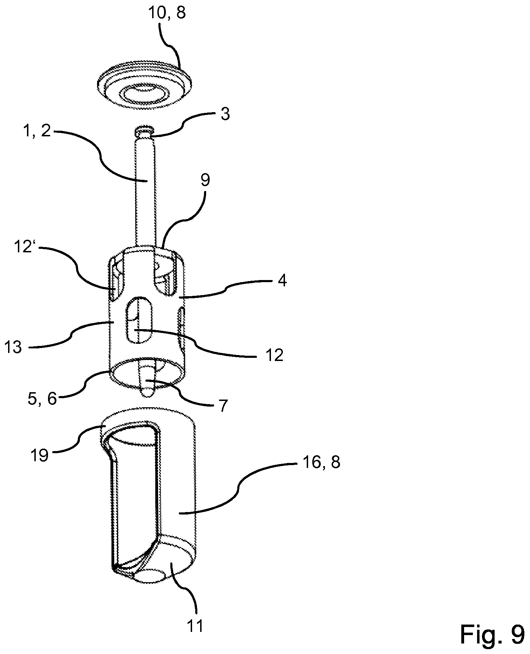

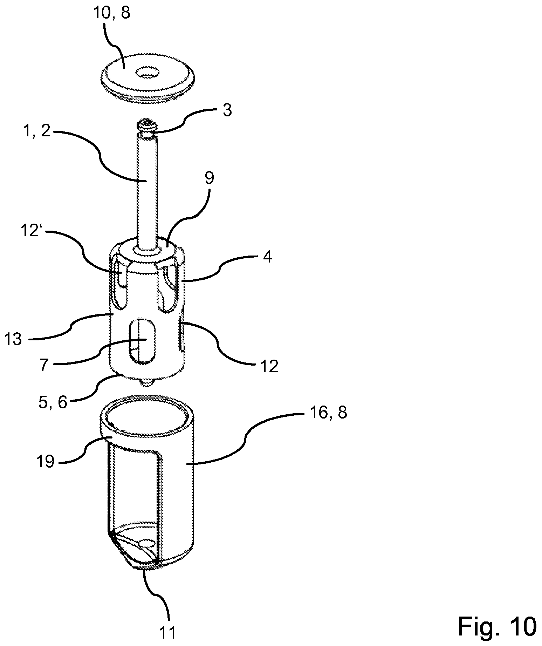

Wie insbesondere aus den Figuren 9 und 10 hervorgeht, ist die Schutzeinrichtung 8 in dem hier dargestellten Ausführungsbeispiel zweiteilig ausgebildet, weist nämlich einen Boden 10 und ein Zylinderelement 16 auf. Der Boden 10 und das Zylinderelement 16 können form- und/oder kraftschlüssig bzw. stoffschlüssig miteinander verbunden sein, beispielsweise geschraubt, geklemmt, geklebt, ultraschallverschweißt etc. Der Boden 10 der Schutzeinrichtung 8 kann dabei in vorteilhafter Weise zumindest bereichsweise flächig auf dem Boden 9 des Kopfes 4 aufliegen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Bearbeitungseinrichtung
- 2: Schaft
- 3: Anschlussbereich
- 4: Kopf
- 5: distaler Rand (Kopf)
- 6: Wirkbereich
- 7: Lagerelement
- 8: Schutzeinrichtung
- 9: Boden (Kopf)
- 10: Boden (Schutzeinrichtung)
- 11: distales Ende (Schutzeinrichtung)
- 12, 12': Öffnung (Kopf)
- 13: Seitenwand
- 14: Fluidkanal
- 15: Fluidöffnung
- 16: Zylinderelement
- 17: Schräge
- 18: Eckbereich
- 19: Rand

## Patentansprüche

1. Chirurgisches Instrument zur Anwendung in der Dentalchirurgie, mit einer Bearbeitungseinrichtung (1) und einer Schutzeinrichtung (8), wobei die Bearbeitungseinrichtung (1) einen Schaft (2) mit einem Anschlussbereich (3) und einem hohlzylinderförmigen Kopf (4) aufweist, wobei an einem distalen Rand (5) des Kopfes (4) ein Wirkbereich (6) zum spanenden oder schleifenden Bearbeiten von Knochen, ausgebildet ist, wobei die Schutzeinrichtung (8) den Kopf (4) zumindest bereichsweise umgibt, wobei die Schutzeinrichtung (8) den Kopf (4) derart umgibt, dass lediglich ein Kreisbogen des distalen Randes (5) als Wirkbereich (6) dient und wobei die Schutzeinrichtung (8) mit einem sich zumindest bereichsweise innerhalb des Kopfes (4) erstreckenden Lagerelement (7) der Bearbeitungseinrichtung (1) drehbar gekoppelt ist.

2. Chirurgische Instrument nach Anspruch 1, **dadurch gekennzeichnet, dass** sich das Lagerelement (7) in Axialrichtung, insbesondere von dem Boden (9) des Kopfes (4), zumindest bis zu dem distalen Rand (5) des Kopfes (4) erstreckt.

3. Chirurgische Instrument nach Anspruch 1 oder 2, **dadurch gekennzeichnet, dass** sich das Lagerelement (7) in Axialrichtung bis über den distalen Rand (5) des Kopfes (4) hinweg erstreckt.

4. Chirurgische Instrument nach einem der Ansprüche 1 bis 3, **dadurch gekennzeichnet**, das das Lagerelement (7) zapfenförmig ausgebildet ist.

5. Chirurgische Instrument nach einem der Ansprüche 1 bis 4, **dadurch gekennzeichnet, dass** die Schutzeinrichtung (8) an ihrem distalen Ende (11) mit dem Lagerelement (7) gekoppelt ist.

6. Chirurgische Instrument nach einem der Ansprüche 1 bis 5, **dadurch gekennzeichnet, dass** die Schutzeinrichtung (8) an ihrem distalen Ende (11) geschlossen ausgebildet ist.

7. Chirurgisches Instrument nach einem der Ansprüche 1 bis 6, **dadurch gekennzeichnet, dass** die Schutzeinrichtung (8) an ihrem proximalen Ende mit dem Schaft (2) drehbar gekoppelt ist.

8. Chirurgische Instrument nach einem der Ansprüchen 1 bis 7, **dadurch gekennzeichnet, dass** die Schutzeinrichtung (8) über ein Gleitlager (17) und/oder ein Kugellager und/oder ein Wälzlager mit dem Lagerelement (7) und/oder dem Schaft (2) drehbar gekoppelt ist.

9. Chirurgische Instrument nach einem der Ansprüche 1 bis 8, **dadurch gekennzeichnet, dass** an dem Lagerelement (7) mindestens eine Fluidöffnung (15) ausgebildet ist, die mit einem Fluidkanal (14) in Verbindung steht, wobei sich der Fluidkanal (14) von dem Anschlussbereich (3) durch den Schaft (2) und das Lagerelement (7) hindurch erstreckt.

10. Chirurgische Instrument nach einem der Ansprüche 1 bis 9, **dadurch gekennzeichnet, dass** die Schutzeinrichtung (8) aus Kunststoff oder Metall hergestellt ist.

11. Chirurgische Instrument nach einem der Ansprüche 1 bis 10, **dadurch gekennzeichnet, dass** die Schutzeinrichtung (8) transparent ausgebildet ist.

12. Chirurgische Instrument nach einem der Ansprüche 1 bis 11, **dadurch gekennzeichnet, dass** in der Seitenwand (13) des Kopfes (4) zumindest eine Öffnung (12) ausgebildet ist.

## Claims

1. Surgical instrument for use in dental surgery, having a processing device (1) and a protection device (8) wherein the processing device (1) has a shaft (2) having a connection region (3) and a hollow-cylindrical head (4), wherein at a distal edge (5) of the head (4) an active region (6) for cutting or grinding processing of bones is constructed, wherein the protection device (8) surrounds the head (4) at least in regions, wherein the protection device (8) surrounds the head (4) in such a manner that only a circular arc of the distal edge (5) acts as an active region (6) and wherein the protection device (8) is rotatably coupled to a bearing element (7) of the processing device (1), which bearing element (7) extends inside the head (4) at least in regions.

2. Surgical instrument according to claim 1, **characterised in that** the bearing element (7) extends in an axial direction, in particular from the base (9) of the head (4), at least as far as the distal edge (5) of the head (4).

3. Surgical instrument according to claim 1 or claim 2, **characterised in that** the bearing element (7) extends in an axial direction over the distal edge (5) of the head (4).

4. Surgical instrument according to any one of claims 1 to 3, **characterised in that** the bearing element (7) is constructed in a journal-like manner.

5. Surgical instrument according to any one of claims 1 to 4, **characterised in that** the protection device (8) is coupled at the distal end (11) thereof to the bearing element (7).

6. Surgical instrument according to any one of claims 1 to 5, **characterised in that** the protection device (8) is constructed to be closed at the distal end (11) thereof.

7. Surgical instrument according to any one of claims 1 to 6, **characterised in that** the protection device (8) is rotatably coupled at the proximal end thereof to the shaft (2).

8. Surgical instrument according to any one of claims 1 to 7, **characterised in that** the protection device (8) is rotatably coupled to the bearing element (7) and/or the shaft (2) by means of a plain bearing (17) and/or a ball bearing and/or a roller bearing.

9. Surgical instrument according to any one of claims 1 to 8, **characterised in that** there is constructed in the bearing element (7) at least one fluid opening (15) which is connected to a fluid channel (14), wherein the fluid channel (14) extends from the connection region (3) through the shaft (2) and the bearing element (7).

10. Surgical instrument according to any one of claims 1 to 9, **characterised in that** the protection device (8) is produced from plastics material or metal.

11. Surgical instrument according to any one of claims 1 to 10, **characterised in that** the protection device (8) is constructed in a transparent manner.

12. Surgical instrument according to any one of claims 1 to 11, **characterised in that** at least one opening (12) is constructed in the side wall (13) of the head (4).

## Revendications

1. Instrument chirurgical destiné être utilisé en chirurgie dentaire, avec un dispositif d'usinage (1) et un dispositif de protection (8), le dispositif d'usinage (1) comportant un manche (2), doté d'une zone de raccordement (3), et une tête (4) en forme de cylindre creux, une zone opérationnelle (6) destinée à l'usinage d'os par enlèvement de matière ou par meulage étant constituée sur un bord (5) distal de la tête (4), le dispositif de protection (8) entourant au moins par tronçons la tête (4), le dispositif de protection (8) entourant la tête (4) de telle sorte que seul un arc de cercle du bord (5) distal sert de zone opérationnelle (6), et le dispositif de protection (8) étant couplé en rotation à un élément de support (7) du dispositif d'usinage (1) qui s'étend au moins par tronçons à l'intérieur de la tête (4).

2. Instrument chirurgical selon la revendication 1, **caractérisé en ce que** l'élément de support (7) s'étend dans la direction axiale, en particulier à partir du bas (9) de la tête (4) au moins jusqu'au bord (5) distal de la tête (4).

3. Instrument chirurgical selon la revendication 1 ou 2, **caractérisé en ce que** l'élément de support (7) s'étend dans la direction axiale jusque par-dessus le bord (5) distal de la tête (4).

4. Instrument chirurgical selon l'une des revendications 1 à 3, **caractérisé en ce que** l'élément de support (7) est constitué en forme de de tenon.

5. Instrument chirurgical selon l'une des revendications 1 à 4, **caractérisé en ce que**, au niveau de son extrémité distale (11), le dispositif de protection (8) est couplé à l'élément de support (7).

6. Instrument chirurgical selon l'une des revendications 1 à 5, **caractérisé en ce que**, à son extrémité distale (11), le dispositif de protection (8) est constitué de façon fermée.

7. Instrument chirurgical selon l'une des revendications 1 à 6, **caractérisé en ce que**, à son extrémité proximale, le dispositif de protection (8) est couplé en rotation au manche (2).

8. Instrument chirurgical selon l'une des revendications 1 à 7, **caractérisé en ce que** le dispositif de protection (8) est couplé en rotation à l'élément de support (7) et/ou au manche (2) par le biais d'un palier lisse (17) et/ou d'un roulement à billes et/ou d'un palier à roulement.

9. Instrument chirurgical selon l'une des revendications 1 à 8, **caractérisé en ce que**, sur l'élément de support (7), il est constitué au moins une ouverture pour fluide (15) qui est en liaison avec un canal pour fluide (14), le canal pour fluide (14) s'étendant à partir de la zone de raccordement (3) à travers le manche (2) et l'élément de support (7).

10. Instrument chirurgical selon l'une des revendications 1 à 9, **caractérisé en ce que** le dispositif de protection (8) est réalisé en matière plastique ou en métal.

11. Instrument chirurgical selon l'une des revendications 1 à 10, **caractérisé en ce que** le dispositif de protection (8) est constitué de façon transparente.

12. Instrument chirurgical selon l'une des revendications 1 à 11, **caractérisé en ce qu'**au moins une ouverture (12) est constituée dans la paroi latérale (13) de la tête (4).
